Europäisches Patentamt

European Patent Office

Office européen des brevets

(19)

(11) Publication number: **0 429 218 A1**

# EUROPEAN PATENT APPLICATION

(21) Application number: 90312189.5

(22) Date of filing: 07.11.90

(51) Int. Cl.5: **G01N 33/53**

(30) Priority: 17.11.89 US 438823

(43) Date of publication of application:
29.05.91 Bulletin 91/22

(84) Designated Contracting States:
CH DE ES FR GB IT LI SE

(71) Applicant: BAXTER INTERNATIONAL INC.
One Baxter Parkway
Deerfield, IL 60015(US)

(72) Inventor: Kirchick, Howard J.
3903 West Sailboat Drive
Cooper City, Florida 33026(US)

(74) Representative: MacGregor, Gordon et al
ERIC POTTER & CLARKSON St. Mary's Court
St. Mary's Gate
Nottingham, NG1 1LE(GB)

(54) Nonhuman primate antibodies for use as immunoglobulin assay positive control.

(57) A nonhuman primate antibody based positive control for use with an immunoglobulin assay. This nonhuman primate antibody is produced by immunizing a nonhuman primate with an antigen and isolating antibodies produced by said nonhuman primates to said antigen. Naturally derived antigens or those produced by genetic engineering or synthetic chemistry can be used.

EP 0 429 218 A1

Xerox Copy Centre

# NONHUMAN PRIMATE ANTIBODIES FOR USE AS IMMUNOGLOBULIN ASSAY POSITIVE CONTROL

## BACKGROUND OF THE INVENTION
### 1. Field of the Invention

This invention relates to the use of nonhuman primate antibodies for use as immunoglobulin assay positive control.

### 2. Prior Art

Certain diagnostic assays detect the response of the human immune system to infectious agents by measuring human immunoglobulins in serum or plasma samples. Traditionally, positive control sera are obtained from individuals who have been exposed to the etiological agent which elicits the immune response. Thus, there is a potential for the assay positive control to be infectious. This invention involves the use of antibodies raised against specific antigens in nonhuman primates (e.g. monkeys) for use in the preparation of positive controls for diagnostic assays.

The current practice for the preparation of positive controls for diagnostic assays measuring specific human antibodies is to use serum or plasma from donors who are positive for the presence of the specific antibodies in question. In certain instances, a specific antibody may coexist in the peripheral circulation with an infectious microbe. In such cases, the blood required for the preparation of the positive control will be potentially infectious. For instance, immunoglobulins of the M class (IgM) which are specific for hepatitis B core antigen may be present at the same time as hepatitis B virus in an individual's peripheral circulation. To prevent positive control material prepared from blood drawn from such an individual from being infectious, one must separate the virus particles from the antibody molecules, or one must inactivate the virus without destroying antigenic determinants on the antibody molecule. Both of these procedures are costly and time consuming and neither are easily demonstrated to be completely effective. The practice described herein allows one to produce antibodies to infectious agents in nonhuman primates without infecting the animal. Thus, noninfectious immune serum or plasma can be used for the manufacture of assay positive control.

A second advantage to the practice described herein is the ability to obtain substantial quantities of specific antibodies with a minimum effort. For example, collecting substantial quantities of blood containing M class immunoglobulins specific for hepatitis A virus is extremely difficult because individuals who have these antibodies are usually ill and cannot afford to donate blood. The percentage of apparently healthy individuals who are positive for these antibodies is extremely small and thus, screening for them would be labor intensive and costly. Using the invention described herein, one need only screen a relatively small number of nonhuman primates which have been treated to elicit the desired immune response.

As noted above, positive controls for assays which detect human antibodies to specific antigens are typically human antibodies which bind to the desired specific antigens. These antibodies and antigens are referred to as immunoreactants and are binding partners of each other. Besides their ability to bind to specific antigens, the positive control antibodies, in many cases, must also be recognized by anti-human immunoglobulin antibodies. All of these assays have a step in which the positive control antibody binds to the specific antigen in question. In many of these assays, the positive control antibody is bound by an antibody against human antibodies which is produced in some other animal.

## SUMMARY OF THE INVENTION

This invention relates to the use of nonhuman primate antibodies for use as immunoglobulin assay positive control. In particular, a positive nonhuman primate based control for use in an immunoassay of human immunoreactant comprising an antibody derived from a nonhuman primate to the binding partner of said human immunoreactant is disclosed.

The nonhuman primate antibodies against antigens, either natural or recombinant, are produced by immunizing a nonhuman primate with the antigen.

## DETAILED DESCRIPTION OF THE INVENTION

One practices this invention by immunizing a nonhuman primate with a specific antigen, monitoring the animal for an immune response, and collecting blood during the immune response. The blood is processed to either serum or plasma and may be diluted to an appropriate titer. This material can then be used as the positive control for an assay measuring immune response to the antigen in question. One may want the assay positive control to contain specific subclasses of immunoglobulins, such as IgM antibodies, since some assays are designed to detect a specific subclass of antibody to an antigen (e.g. an IgM anti-HAV assay). In cases such as this, one must monitor the bleeds for class specific antibodies. The antigen used can be a microbial antigen produced using recombinant DNA technology, synthetic chemistry, or may be purified from the microbe. Each of these methods should produce an antigen which will be devoid of infectivity. Thus, when the nonhuman primate is immunized with the antigen, the animal will remain noninfectious.

One can practice this invention because of the high degree of similarity between human and monkey immunoglobulins which is most likely the result of the close phylogenic relationship between the primate families. One way in which this high degree of similarity manifests itself is in the level of recognition of human and monkey immunoglobulins by lower mammals, such as mice, rabbits, goats, and sheep. In other words, antibodies raised in mice against human antibodies bind to both human antibodies and monkey antibodies.

An example of the practice of this invention follows. Rhesus monkeys were immunized with hepatitis B core antigen (HBcAg) which was prepared using recombinant DNA technology. The monkeys were bled periodically and the blood was processed to serum. The serum was then assayed for the presence of IgM antibodies to HBcAg using a class capture assay. Briefly, murine anti-human Igm was immobilized on a solid surface. A dilution of the serum sample was added to the solid surface which resulted in the immobilization of monkey IgM antibodies. HBcAg was then added which bound only to those IgM antibody molecules which were specific for HBcAg. Detection was accomplished with the addition of an enzyme labeled murine anti-HBcAg. The results of these assays indicate that rhesus monkeys mount an IgM immune response to injected HBcAg and that this immune response can be detected in an IgM anti-HBcAg assay which utilizes an anti-human IgM capture antibody. Thus, rhesus monkey serum produced in such a way could serve as a positive control for an IgM anti-HBcAg immunoassay.

Example 1

This example describes a method for preparing a positive control serum for an immunoassay designed to detect IgM antibodies to hepatitis B core antigen (HBcAg) in accordance with the present invention.

A. Preparation of Immunogen

1. HBcAg - a 32 μg/ml stock solution was made by dissolving 320 μg of genetically engineered HBcAg in 10 ml of a 50 mM sodium phosphate, 150 mM sodium chloride, pH 7.5 solution.
2. Vaccine - 10 ml of incomplete Freunds adjuvant was added to the 10 ml of 32 μg/ml HBcAg stock solution. After mixing thoroughly, the resultant vaccine contained 16 μg/ml of HCaAg.

B. Animals

Adult male Rhesus monkeys averaging approximately 9 kg of body weight were used. All animals underwent a physical examination, which included a complete blood count, serum chemistry screen, fecal exam, B virus screen, and SIV screen.

C. Protocol

At least 2 ml of blood was collected from each monkey prior to vaccination to establish a baseline level of IgM antibodies to HBcAg (IgM anti-HBc). Each animal was injected with vaccine by an intramuscular route (arm or leg) such that each animal received HBcAg at 2 μg/kg of body weight. At least 2 ml of blood was collected twice a week from which serum was prepared. The biweekly serum samples were assayed for the presence of IgM anti-HBc. A rise in the titer of IgM anti-HBc was apparent within one to two weeks after vaccination.

When the titer of IgM anti-HBc reached an appropriate level, the animals were bled for maximum recovery until such time as the IgM anti-HBc titer began to fall. Serum was prepared from each of these bleeds and was pooled into a positive control stock pool. Aliquots from the positive control stock pool were diluted to an appropriate level to serve as a positive control in an IgM anti-HBc immunoassay. This was accomplished by diluting the positive control stock pool to a level which matched the positive control in an FDA approved IgM anti-HBc immunoassay.

Example 2

This example describes a method for using nonhuman primate sera containing IgM anti-HBc as a positive control in an IgM anti-HBc radial partition immunoassay. Use of the nonhuman primate positive control serum is applicable to any other type of immunoassay as well.

A. Reagents

1. IgM Class Capture System - A monoclonal anti-human IgM antibody was immobilized on a glass fiber filter paper tab as a complex with a goat antibody to the Fc region of mouse IgG.
2. HBcAg Reagent - Genetically engineered HBcAg was diluted to 50 ng/ml in a pH 7.6 Tris buffer containing NaCl, BSA, a surfactant, and 0.1% sodium azide as preservative.

3. Sample Diluent - A pH 7.6 Tris buffer containing BSA, gelatin, NaCl, a surfactant, and 0.1% sodium azide as preservative.

4. Negative Control - Human serum with no detectable IgM anti-HBc.

5. Positive Control - Nonhuman primate serum containing IgM anti-HBc as a result of immunization with genetically engineered HBcAg. This reagent is prepared as described in Example 1.

6. Anti-HBc Enzyme Conjugate - Fab' fragments of a monoclonal antibody to HBcAg was conjugated to calf intestinal alkaline phosphatase using sulfo-SIAB as the crosslinking reagent.

7. Substrate Wash Solution - 0.39 mM 4-methylumbelliferyl phosphate in a pH 9.0 Tris buffer containing stabilizers, and surfactant with 0.1% sodium azide as preservative.

B. Assay for IgM anti-HBc

Samples and controls are diluted 1:1000 in sample diluent. The controls are assayed in duplicate and the samples may be assayed as singlicates. 50 μl of the diluted samples and controls are pipetted to individual IgM class capture filter paper tabs. Human and nonhuman primate IgM antibodies will bind to the IgM anti-HBc immobilized on the tabs regardless of the specificity of the IgM antibodies. Immediately following the addition of the diluted samples and controls to the class capture tabs, 50 μl of HBcAg Reagent is pipetted to each tab. The HBcAg will bind only to those captured IgM molecules which are specific for HBcAg. The combined incubation time for the diluted sample and controls and the HBcAg Reagent is two minutes. 40 μl of IgM anti-HBc enzyme conjugate is then added to each class capture tab. The enzyme-antibody conjugate will bind to HBcAg, which has bound to the captured HBcAg specific IgM antibody molecules. After three minutes, 70 μl of the substrate wash solution is added to each tab. This reagent serves to initiate a fluorogenic enzyme reaction and to cause the radial partitioning of bound and unbound enzyme-antibody conjugate (U.S. Patent's 4,517,288, 4,786,606, and 4,774,174). The rate of generation of fluorescence in the area of the tab containing the bound conjugate is measured.

To determine whether a sample is considered to be positive or negative for IgM anti-HBc, an assay cutoff value is calculated. Since the nonhuman primate IgM anti-HBc in the positive control is recognized by the anti-human IgM immobilized on the class capture tab, it can be diluted to an appropriate level and be used interchangeably with human IgM anti-HBc as positive control antibody. The assay cutoff in this example is the mean of the negative control values plus 0.25 times the mean of the positive control values. IgM anti-HBc levels within 10% of this cutoff are considered to be indeterminant and require retesting. IgM anti-HBc levels below 10% of this cutoff value are not considered to be clinically significant.

It will be appreciated that the foregoing advantages were achieved in a radial partition immunoassay, but the use of positive nonhuman primate based controls are useful for any immunoassay.

**Claims**

1. A positive nonhuman primate based control for use in an immunoassay.

2. A positive nonhuman primate based control for use in immunoassay of a human immunoreactant comprising an antibody derived from a nonhuman primate to the binding partner of said human immunoreactant.

3. The control of claim 1 wherein said nonhuman primate is a monkey.

4. A positive nonhuman private based control for immunoassay made by the process comprising:
   a) immunizing said nonhuman primate with an antigen, and
   b) collecting antibodies produced by said nonhuman primates to said antigen.

5. The method of claim 4 wherein said antigen is genetically engineered, synthetic, or natural.

European
Patent Office

**EUROPEAN SEARCH
REPORT**

Application Number

**EP 90 31 2189**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CURRENT MICROBIOLOGY. vol. 10, 1984, pages 261-264; B.J. BRAKE et al.: "Antigenic relatedness of immunoglobulins to Legionella pneumophila serogroups 1-6 from humans and the nonhuman primate Macaca fascicularis" <br> * Page 261 * | 1-5 | G 01 N 33/53 |
| A | WO-A-8 910 980 (BIO-RESEARCH LABORATORIES, INC.) <br> * Abstract; page 1, line 1 - page 6, line 23 * | 1-5 | |

TECHNICAL FIELDS
SEARCHED (Int. Cl.5)

G 01 N

The present search report has been drawn up for all claims

| Place of search | Date of completion of search | Examiner |
|---|---|---|
| The Hague | 11 January 91 | HITCHEN C.E. |

CATEGORY OF CITED DOCUMENTS
X : particularly relevant if taken alone
Y : particularly relevant if combined with another
   document of the same catagory
A : technological background
O : non-written disclosure
P : intermediate document
T : theory or principle underlying the invention

E : earlier patent document, but published on, or after
   the filing date
D : document cited in the application
L : document cited for other reasons

&: member of the same patent family, corresponding
   document